# EUROPEAN PATENT APPLICATION

(11) **EP 4 583 115 A1**
(43) Date of publication of application: **09.07.2025**
(21) Application number: 24150619.5
(22) Date of filing: 08.01.2024
(51) Int. Cl.: G16H 10/60

(54) **COMPUTER-IMPLEMENTED METHOD AND PORTAL FOR PROVIDING PATIENT DATA**

(71) Applicant: Siemens Healthineers AG, 91301 Forchheim (DE)
(72) Inventor: Mangesius, Patrick, 6020 Innsbruck (AT); Schabetsberger, Thomas, 6068 Mils (AT)
(74) Representative: Siemens Healthineers Patent Attorneys

(57) **Abstract**

The present invention relates to a computer-implemented method and to a portal for providing data of a patient used in a scheduled appointment with a healthcare service provider, wherein the method comprises the steps of: determining patient data required for the scheduled appointment depending on an appointment type of the scheduled appointment; calculating missing patient data depending on the determined patient data required for the scheduled appointment and depending on available patient data of the respective patient stored in an electronic health record of the patient; determining at least one suitable data input channel for the calculated missing patient data; requesting a transfer of the calculated missing patient data from a terminal device of the patient via the determined data input channel; and transferring by the patient the requested missing patient data via the determined data input channel.

## Description

The present invention relates to a computer-implemented method. The present invention further relates to a portal for providing patient data for a scheduled appointment with a healthcare service provider.

Patients are increasingly engaged in their treatment process, transforming healthcare IT into a personalized, largely connected, and patient empowering infrastructure.

For improving the continuity of healthcare and establishing an effective cooperative healthcare system, shared electronic health records (S-EHR) have been introduced to close the gap between institution-specific patient data and a comprehensive, longitudinal collection of health-related patient data. In addition to the sharing and collecting of patient data, the empowerment of patients to understand their own medical data and to manage their own patient journey forms a key pillar for a cooperative healthcare system with engaged patients.

To fulfill such demands conventional patient portals have been established in many regions and countries around the world to provide patients with entry points and management possibilities to handle their own patient data. Many of these conventional patient portals offer only the functionality of display and exchange of data.

To provide additional functionalities for the patient the provision of smart and intelligent mechanisms is helpful to avoid an information overflow at the patient's side as well as offering digital guidance to the patient through the healthcare system. This is especially true for the management of appointments with healthcare service providers such as physicians. Appointments and interactions with healthcare service providers are essential to help in keeping the patient engaged.

To achieve a maximum benefit of appointments it is crucial for healthcare service providers, that the patient is prepared and that all necessary data for the treatment or consultation of the patient is available to the healthcare service provider at the time of the scheduled appointment. This helps to avoid additional research, examination, and patient interviews or referrals. Additionally, the healthcare professional benefits from the possibility of preparing himself prior of the appointment with the patient based on the available patient data.

Especially in the digital age, where online appointment booking has lowered the barrier of getting in contact with a healthcare service providers an invalid, insufficient patient data set when conducting a treatment of the patient during the scheduled appointment are cost drivers for digitally enhanced healthcare systems and institutions.

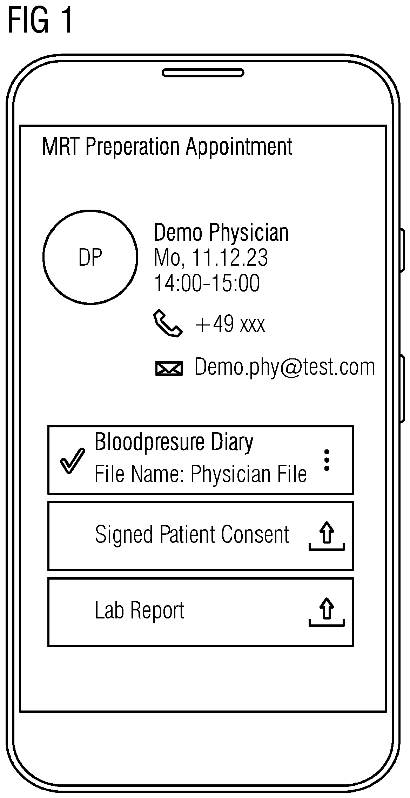

An example of a conventional way for providing patient related information prior to an appointment from a patient's perspective is illustrated in Fig.1. In this example, the healthcare service provider is a physician. A patient has the option to upload specific documents for a scheduled appointment with the physician. To allow the collection of necessary data, conventional patient portals provide upload possibilities of documents.

However, the gathering of missing patient data by conventional patient portals does not take into account an already available set of patient data of the patient and does not guide the patient to provide all necessary data required for a successful treatment or consultation at the scheduled appointment with the healthcare professional. This may also lead to a management overhead on the professional side, an unwanted and confusing duplication of patient data and an unnecessary and redundant reexamination of already available patient data by the healthcare service provider. Moreover, insufficient or not complete patient data may lead to an erroneous treatment or consultation of the patient by the healthcare service provider during the appointment.

Against this background, the object addressed by the present invention is to provide a method and a patient portal that allow to efficiently gather patient data of a patient for a scheduled appointment with a healthcare service provider.

This object is achieved by a computer-implemented method comprising the features of claim 1 and/or by a patient portal comprising the features of claim 11.

The invention provides according to a first aspect a computer-implemented method for providing data of a patient used in a scheduled appointment with a healthcare service provider, wherein the method comprises the steps of: determining patient data required for the scheduled appointment depending on an appointment type of the scheduled appointment; calculating missing patient data depending on the determined patient data required for the scheduled appointment and depending on available patient data of the respective patient stored in an electronic health record (EHR) of the patient; determining at least one suitable data input channel for the calculated missing patient data; requesting a transfer of the calculated missing patient data from a terminal device of the patient and/or from a terminal device of another healthcare service provider via the determined data input channel; and receiving from the terminal device of the patient and/or from the terminal device of the other healthcare service provider the requested missing patient data transferred via the determined data input channel.

The computer-implemented method can be performed by a program executed on a server of a portal forming part of a healthcare system.

The present invention provides according to a further aspect a computer program product having a stored program code adapted to execute a computer-implemented method for providing data of a patient used in a scheduled appointment with a healthcare service provider, wherein the method comprises the steps of: determining patient data required for the scheduled appointment depending on an appointment type of the scheduled appointment; calculating missing patient data depending on the determined patient data required for the scheduled appointment and depending on available patient data of the respective patient stored in an electronic health record (EHR) of the patient; determining at least one data input channel for the calculated missing patient data; requesting a transfer of the calculated missing patient data from a terminal device of the patient and/or from a terminal device of another healthcare service provider via the determined data input channel; and receiving from the terminal device of the patient and/or from the terminal device of the other healthcare service provider the requested missing patient data transferred via the determined data input channel.

The program code can be stored on a machine-readable data storage medium, such as a CD, DVD, USB stick or the like.

The invention provides according to a further aspect a patient portal comprising a server adapted to perform the computer-implemented method.

A technical idea underlying the present invention is to determine automatically missing patient data prior to a scheduled appointment with a healthcare service provider and to trigger actively the completion of the required patient data by the patient and/or by another healthcare service provider participating in the system.

A healthcare service provider is any person or institution providing a healthcare service including a physical treatment, a diagnostic investigation or a consultation of a patient. A healthcare service provider can be a healthcare professional such a physician or a health instructor or an institution such as a laboratory.

Advantageous configurations and developments emerge from the further dependent claims and from the description with reference to the figures of the drawings.

In a possible embodiment of the computer-implemented method, the data input channel comprises a document submission channel, a patient input channel and/or a monitoring device input channel. This offers a variety of different information submission channels to the user, in particular to the patient, to submit efficiently different kinds of information in relative short time. Further structured or unstructured data can be submitted or transferred to the platform or portal. Different data input channels can be offered and selected by the patient to submit or transfer the required data taking into account the available technical equipment of the patient.

In a possible embodiment of the computer-implemented method, the missing patient data submitted via the derived data input channel is stored in the electronic health record (EHR) of the patient. The electronic health record (EHR) of the patient can be stored in database of the patient portal. Consequently, the electronic health record is always up-to date and further inquiries for missing data are avoided.

In a possible embodiment of the computer-implemented method, at least one data provision task is generated automatically for a patient and/or for another healthcare service provider to provide the missing patient data of the patient required for the scheduled appointment. This facilitates the management and handling of patient data for the patient using the portal. Further, the data provision task may set a deadline to the patient which can be loaded to an electronic calendar of the user or patient. In this way a user, in particular a patient, can be reminded to fulfill the set task in time. The generated data provision task can be transmitted in a message to a terminal device of the patient and/or to a terminal device of the healthcare service provider.

In a possible embodiment of the computer-implemented method, the calculated missing patient data comprises missing patient data available to the patient and/or missing patient data to be provided by another healthcare service provider. This enhances collaboration between patients and healthcare service providers. Moreover, the portal participant being in possession of the required data can be directly addressed to provide the required data. This way, the required data can be provided more efficiently and in shorter time to the portal.

In a possible embodiment of the computer-implemented method, a data provision task is triggered automatically if the calculated missing patient data comprises missing patient data to be provided by another healthcare service provider. This increases automation of the process and facilitates the handling for a participant or user, in particular for a healthcare provider or patient.

In a possible embodiment of the computer-implemented method, the other healthcare service provider system provides the missing patient data via a professional data input channel in response to the triggered data provision order task. The professional data input channel can comprise a high bandwidth to increase the data transmission rate of high volume data, in particular image data. Further, the professional data input channel may comprise additional safety mechanisms for data protection, in particular data encryption mechanisms, to protect patient data from unwanted access by third parties.

In a possible embodiment of the computer-implemented method, the other healthcare service provider evaluates the data provision order task to schedule automatically an appointment with the respective patient for generation of the missing patient data if necessary. The scheduled appointment can involve a personal meeting or a video conference. The automatic scheduling of an appointment facilitates the handling for the entrusted healthcare service provider and speeds up the process.

In a possible embodiment of the computer-implemented method, the appointment is setup and booked by a participating user including the patient or the healthcare service provider. Different available appointment dates and appointment times may be proposed automatically to the patient. The patient can choose a fitting appointment date and time that also matches a time slot available for the healthcare service provider.

In a further possible embodiment of the computer-implemented method, the appointment is setup automatically in response to an observed event. The observed event can comprise a critical medical condition of the patient. This reduces significantly risks for patients having a severe health condition and also allows to prioritize critical patients when scheduling an appointment. A critical medical condition can be determined or detected automatically by a health monitoring device or determined by an observing healthcare professional visiting the patient at home or in a room of a hospital. The health monitoring device is configured to monitor at least one health related parameter of the patient such as a heartbeat rate or a body temperature of the patient.

Where appropriate, the above-mentioned configurations and developments can be combined implementations can be combined with each other as desired, as far as this is reasonable. Further possible configurations, developments and implementations of the invention also include combinations, which are not explicitly mentioned, of features of the invention which have been described previously or are described in the following with reference to the embodiments. In particular, in this case, a person skilled in the art will also add individual aspects as improvements or supplements to the basic form of the present invention.

The present invention is described in greater detail in the following on the basis of the embodiments shown in the schematic figures of the drawings, in which:
- Fig.1: illustrates an option of uploading patient data in a conventional patient portal;
- Fig.2: illustrates a flow chart of a possible exemplary embodiment of a computer-implemented method according to an aspect of the present invention;
- Fig.3: illustrates a possible data storage structure of electronic health records and data input channels used by a computer-implemented method and portal according to the present invention;
- Fig.4: illustrates a possible data structure of data required for an appointment used by a computer-implemented method and portal according to the present invention.

The appended drawings are intended to provide further understanding of the embodiments of the invention. They illustrate embodiments and, in conjunction with the description, help to explain principles and concepts of the invention. Other embodiments and many of the advantages mentioned become apparent in view of the drawings. The elements in the drawings are not necessarily shown to scale.

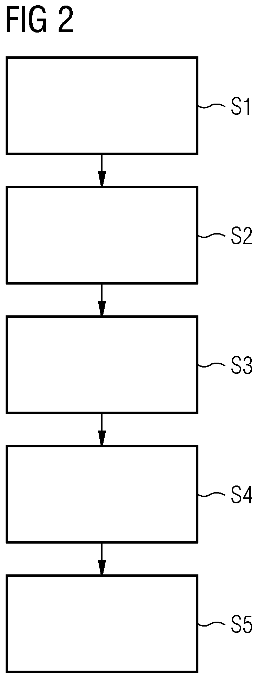

As illustrated in the flow chart of Fig. 2 the computer-implemented method for providing data of a patient used in a scheduled appointment with a healthcare service provider comprises in a possible embodiment several main steps S.

In a first step S1, patient data required for the scheduled appointment with the healthcare service provider (HSP) is determined depending on an appointment type of the scheduled appointment.

The appointment type depends on the service provided by the healthcare service provider. Each healthcare service providers may define different services with associated appointment types and with associated required patient data necessary to perform the respective service by the healthcare service provider during the appointment. The appointment comprises an adjustable time slot ranging from a start time to a stop time. The length of the assigned and booked time slot depends in a possible implementation on the appointment type of the scheduled appointment.

For each appointment type a guideline or a checklist can indicate the patient data or patient data set required as a basis for performing the respective appointment with the healthcare service provider. These guidelines or checklists can be managed by a data management system and distributed to the healthcare service providers. A healthcare service provider can also define or configure individually the patient data required for an appointment with him.

In a possible implementation of the computer-implemented method, the length of the time slot can be re-adjusted depending on the available patient data.

The appointment can be a personal appointment at the location of the healthcare service provider or at the home or room of the patient. The appointment takes place in a scheduled time slot booked by the patient himself or booked by a consulting healthcare service provider, in particular by a healthcare professional such as a doctor or nurse. The appointment can also comprise a virtual online appointment of the patient with one or more healthcare service providers.

The setup of an appointment can be initiated or triggered by a patient or by another participating user of the patient portal. The setup of the appointment can also be triggered by an observed event. The occurrence of an event such as a critical condition of the patient can be detected or determined by a health monitoring device. For instance if a measured blood pressure of a monitored patient exceeds a predefined threshold an appointment of the affected patient with a suitable healthcare service provider such as a cardiologist can be triggered automatically. The occurrence of an event can also be determined by a healthcare professional such as a physician or a nurse observing the patient or visiting the patient in his home.

The scheduled appointment and the date and booked time slot can be notified to the patient. The scheduled appointment can be displayed on a display unit of a user interface of a terminal device or of a user equipment of the patient along with an identification or along with a list of the determined patient data required for the respective appointment.

In a further step S2, missing patient data is calculated depending on the patient data required for the scheduled appointment determined in step S1 and depending on available patient data of the respective patient stored in an electronic health record (EHR) of the patient. Available patient data of the patient stored in the electronic health record (EHR) of the patient or stored otherwise for the patient in a memory or database can be compared with the patient data required for the scheduled appointment to determine automatically still missing patient data of the respective patient.

The calculation can be performed by a processor of a server of the patient portal. Available patient data sets of different types with associated time stamps TS can be evaluated to identify missing data sets or missing data records.

The validity of a found data set of an EHR record can be checked automatically. For example a time stamp TS may indicate that a data record of the patient retrieved from the EHR database is too old (has expired) and is no longer valid for further treatment of the patient.

For instance, the consultation or treatment of a patient by an orthopedist as a first healthcare service provider during a scheduled appointment may require image data of the patient. If this image data is not available to the patient or is too old it is necessary to entrust a radiologist as a second healthcare service provider to generate the required up to date image data in advance to the scheduled appointment with the orthopedist.

The patient data required for an appointment with a healthcare service provider such as an orthopedist can be defined by the respective healthcare service provider or derived from available guidelines or checklists. An orthopedist can configure the necessary patient data set requested by him to perform his service (e.g. consultation service). The required patient data may comprise image data of the affected region of the patient's body and other data such as health insurance data or other health related data (e.g. concerning allergies or vaccinations of the patient).

To optimize the data collection and reduce management overhead on the professional side an algorithm can determine the necessary data based on the requirements of the appointment type of the scheduled appointment as well as the already existing patient data in the patient's EHR.

In a further step S3, at least one suitable data input channel for a data transfer of the calculated missing patient data is determined. A suitable data input channel can be determined depending on the data source and/or depending on a form or data format of the missing patient data to be transferred. A suitable data input channel can be determined in step S3 manually by the patient or healthcare professional or automatically by a device. The suitable data input channel can be determined in a possible embodiment by a server of the patient portal.. The system calculates the missing patient data set in step S2 and can determine or derive subsequently available possibilities of data transfer, i.e. suitable data input channels for the transfer of the missing patient data from a terminal device of the patient and/or from a terminal device of another healthcare service provider to a target device . A suitable data input channel can be determined or can be selected from a group of available data input channels manually or automatically depending on the type or volume of the missing patient data and/or depending on the type of the data source. The data input channel or data transfer channel can be determined automatically using predefined channel determination rules. These channel determination rules can specify data input channels to be used for certain patient data types, patient data formats and/or data source types.

The data input channels can comprise document submission channels, patient input channels and/or monitoring device input channels.

The at least one suitable data input channel determined in step S3 can be notified to a user or healthcare professional in a message sent to a terminal or portable user equipment of the patient or healthcare professional. The message can include a recommendation which of the available data input channels is most suited for the data transfer of the requested patient data.

A document submission channel can be configured such that it is suitable or adapted to submit documents, in particular text documents or text files. A document submission channel can be used by the patient to transfer or submit already existing documents, in particular reports, summaries and templates from a terminal device of the patient to a target device. The target device can comprise a server of the patient portal or a terminal device of a healthcare service provider. Based on the document type of the document the system is aware what data can be expected to be extracted from the respective document. The documents can comprise for example a Laboratory Report (forming a first document type) or a Discharge Letter (forming a second document type). It is possible to define different types of documents used in the treatment process. Those documents originate mainly from previous appointments of the patient with other healthcare service providers, however, they are not yet registered or known to the portal of the healthcare system. An example for such a document is a discharge letter handed over previously to the patient. This letter can be scanned by the patient and transmitted as a PDF file via an electronic data input channel.

A patient input channel can be configured such that it is adapted to collect patient data based on patient's data input by the patient himself or input by a healthcare service provider into a user interface of a terminal device. A patient input channel can involve use of forms and patient questionnaires. These forms or input masks can be displayed on a display unit of a user interface of a terminal device of the patient or healthcare service provider. The patient input channel is adapted to collect structured and unstructured data based on the patient's input (e.g. Medication Diaries, Pain/Blood Diaries, etc.).The collected patient data can be transferred via the patient input channel to a target device comprising the server of the patient portal or comprising a terminal device of a healthcare service provider.

A monitoring device input channel is configured such that it is adapted to collect patient data from one or more connected health monitoring devices. The health monitoring device can in a possible embodiment be located in the vicinity of the observed patient (e.g. a camera or microphone).The health monitoring device can also be attached to the skin of the patient body (e.g. an electrode).The health monitoring device further can be inserted or implanted into the body of the monitored patient.

One or more monitoring device input channels can be used for collecting periodically information or data samples from connected devices, in particular from home monitoring devices, and/or other systems. This allows an indirect gathering of patient data being either directly related or not related to an upcoming scheduled appointment (e.g. patient weight data, patient blood pressure data, etc.).The monitoring device input channel can transfer the collected patient data to a target device comprising a server of the patient portal or comprising terminal device of a healthcare service provider.

The different data input channels can be used to populate the underlying data structure of the shared electronic health record (EHR) of the patient. The data structure of the shared electronic health record can be commonly stored and managed in accordance with interoperability standards such as HL7 FHIR, IHE XDS/XDS-I or DICOM.

The data input channels can comprise different kinds of data channels including electronic input channels specified by unique network addresses such as email addresses. The patient data transferred through an electronic data input channel can comprise data files, in particular text files (e.g. PDF files) or image files, in different file formats. These data formats can comprise also machine-readable data formats. The collected patient data can also be preprocessed and can comprise discrete data generated by sensors or home monitoring devices including data samples or measurement parameters.

In a further step S4, a transfer of the calculated missing patient data is requested from the patient and/or from another healthcare service provider via a determined suitable data input channel. The data transfer request can be sent in a request message to a terminal device of the patient and/or to a terminal device of the healthcare service provider. The transmitted request message can indicate or propose a suitable data input channel as determined in step S3 for performing a data transfer of the missing patient data calculated in step S2.

In a possible embodiment, a data provision task is generated automatically to provide the determined missing patient data. The information and patient data that are required for the scheduled appointment with the healthcare service provider can be requested directly as a predefined set, independent of the information and patient data stored in the electronic health record (EHR) of the patient EHR. The data provision task can comprise an associated deadline for fulfillment, i.e. a deadline for providing the requested patient data

For example if the evaluation of the appointment type of the scheduled appointment with the healthcare service provider (e.g. orthopedist) and the available patient data stored in the shared electronic health record (EHR) of the patient has the result that relevant patient data is still missing (e.g. image data) a task for the patient can be generated automatically. This data provision task can instruct the patient to perform the necessary actions to get the missing patient data and to transfer the missing patient data via a determined suitable data input channel before the date of the scheduled appointment. The data provision task may propose one or more suitable data input channels for the data transfer of the missing patient data.

The data provision task set for the user or patient can be transmitted in a message to a portable user equipment or terminal device of the patient and be displayed to him on a user interface. The data provision task can e.g. be pushed in a message to the terminal device of the user or patient. The transmitted message including the data provision task may include links or recommendations for suitable healthcare service providers capable to generate the requested missing patient data such as missing image data. The patient can contact an indicated healthcare service provider such as a radiologist listed in the received data provision task to set up an appointment with him to generate the missing patient data. This can lead to the generation of a further data provision task for the patient issued by the radiologist specifying for the patient what patient data is requested by the radiologist for the diagnostic investigation of the patient.

After the patient has fulfilled all data provision tasks set by the system on behalf of the radiologist the diagnostic service offered by the radiologist takes place and the missing patient image data is generated and forwarded to a user equipment or terminal device of the patient.

In a further step S5, the requested missing patient data is received from a terminal device or user equipment of the patient via the data input channel as indicated in the request message of step S4. The requested missing patient data can be received in step S5 by a server of the patient portal and can then be used for updating an electronic health record (EHR) of the patient which can be stored in central or distributed database of the patient portal.

The missing patient data transferred via the determined data input channel is stored in the electronic health record (EHR) of the patient to update the electronic health record (EHR) of the patient. The electronic health record (EHR) of a patient can be stored in a central database of the system or portal and/or can be stored in distributed local data storages of health service providers or healthcare professionals and/or in a local data storage of a terminal device of the patient. The missing patient data can also be transferred to the patient portal directly by the entrusted healthcare service provider (e.g. radiologist).The electronic health record (EHR) of the patient is updated with the transferred missing patient data. The health service provider planned to perform the health service during the scheduled appointment gets access to the updated electronic health record (EHR) of the patient.

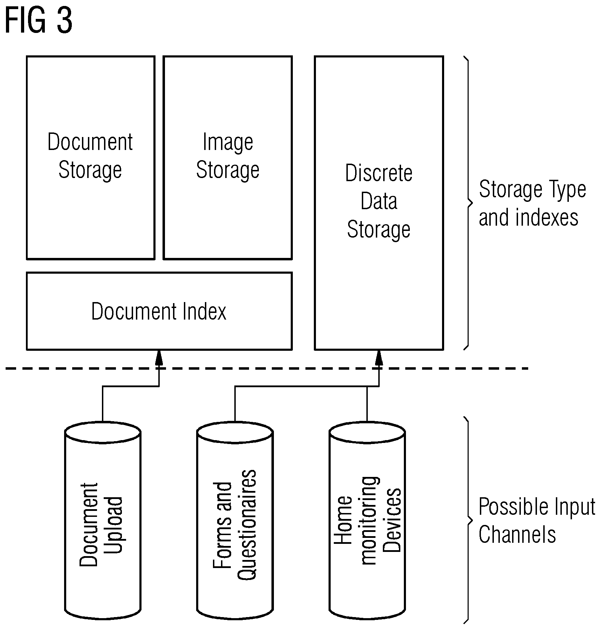

The data structure of the electronic health record (EHR) and possible data input channels can be depicted as illustrated in Fig.3.

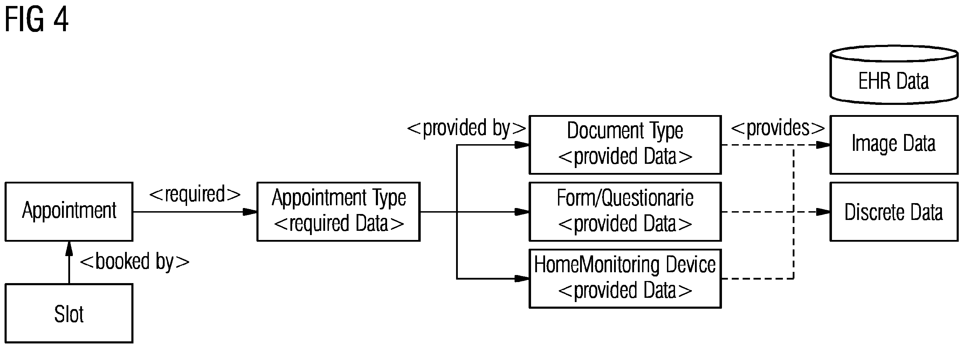

To allow an automated assignment of required patient data for an appointment a data structure as depicted in Fig. 4 can be employed to automatically detect missing patient data and request the missing patient data through the data input channels. The pre-requirement of the data is transformed into a data request notified in a task, which can be fulfilled by the patient or another healthcare service provider or any other participating user through the adequate data input channel. The electronic health record (EHR) data of a patient stored in a database of the patient portal can comprise image data, audio data and discrete data. The discrete data can comprise parameters or data samples related to the health condition of the patient, e.g. body temperature or blood pressure values.

A suitable data input channel can be selected and offered to the user of the patient portal to populate the missing patient data. Through a rule-based configuration the data input channels can be automatically determined and selected without administrative overhead in the management of eHealth or related portals.

The missing patient data calculated in step S2 may also comprise patient data not available or not yet available to the patient but available by making use of medical services of other healthcare service providers. For instance if the missing patient data comprises image data a diagnostic service of a radiologist is required to generate the missing image data of the patient.

In a preferred embodiment, a data provision order task is triggered automatically if the calculated missing patient data comprises missing patient data which can be provided by one or more other healthcare service providers. The healthcare service provider such as a radiologist can provide the missing patient data via a data input channel, in particular via a professional data input channel with a high bandwidth or data transfer rate and/or with an applied security mechanism, to a server of the portal in response to the received task including the data provision order. The missing patient data provided by the healthcare service provider via the professional data input channel can also be stored in the electronic health record (EHR) of the patient, in particular in a database of the patient portal.

In a possible embodiment of the computer-implemented method the healthcare service provider can evaluate the received data provision task to check whether the requested missing patient data is already available in a local storage of the healthcare service provider or not. If the data is not available the missing patient data has to be generated by the healthcare service provider. In case that the patient data requested in the data provision task is not yet available but has to be generated for the respective patient the system can schedule automatically an appointment for the respective patient at another healthcare service provider for generation of the missing patient data by the respective healthcare service provider.

Appointments can in addition to the provision of a patient/end user based data input channel require the provision of a professional data input channel (e.g. Laboratory examinations, image based treatment, etc.) with a high bandwidth and/or comprising a data security mechanism (such as encryption). In this case, the healthcare system automatically triggers a task with an order for another healthcare service provider such as a radiologist, which recursively leads to an appointment selection based on the channels prerequisite. This can trigger in turn tasks directed towards image based diagnostic systems such as modalities, Laboratory Information Systems (LIS) or Radiology Information Systems (RIS) .

Once the appointment is in place, the gathered and prepared patient data is presented to the healthcare professional such as a physician (e.g. orthopedist) on a display of a terminal. The presentation of the data can be done through several ways and applications including an eHealth Patient Chart. This allows the healthcare professional to have all the required data visualized in one place and to guide the patient through the process in an efficient manner.

If a patient does not fulfill a data provision task set for him by the system on behalf of a healthcare service provider an already scheduled appointment for the patient with the respective healthcare service provider can be automatically rescheduled to a later date. This gives the patient more time to get the still missing patient data and avoids conduction of a not meaningful appointment with the healthcare service provider. This makes the process more efficient.

After the treatment during the scheduled appointment, the patient can further interact through the healthcare journey using the patient portal. The information in place, outcome and gathered information allow a seamless creation of follow-up actions and appointments as well as the engagement between healthcare providers and the patient.

In the first step S1, of the computer-implemented method shown in the flow chart of Fig.1 patient data required for the scheduled appointment is determined depending on an appointment type of the scheduled appointment.

The appointment between the patient and the healthcare service provider, in particular a healthcare professional, can be scheduled after identification of a health problem of the patient and a subsequent identification of a suitable specialist.

A reason for scheduling an appointment with a healthcare professional or expert can be relevant for the identification or determination of the patient data required for the respective appointment. In a possible embodiment the appointment type is determined depending on an identified or assumed health problem of the patient or depending on other reasons stated by the patient. These reasons can comprise a desired check of the whole body or body portions of the patient or a check of specific body functions of the patient by a specialized healthcare service provider. In a possible embodiment the patient or a healthcare professional can input a specific reason for scheduling an appointment into a user interface of a terminal.

Initially a health problem of a patient can be assessed. This can be done based on questions and automated interviews to collect the patient's symptoms. Previous illnesses and risk factors are derived from the patient's electronic health record (EHR)and cross-linked with the answers provided by the patient. This allows the suggestion of a treatment path for treating the patient.

Based on the treatment path suitable specialists, physicians and treatment experts can be identified as healthcare service providers. This identification includes the speciality of the expert required to cover the respective treatment step within the treatment path and the current location of the patient. Specialists located in vicinity of the patient are preferred in the appointment evaluation.

Source data for the available services and availabilities of healthcare service provider are commonly stored in Health Care Provider Directories (HPD) or Care Service Directories (CSD). Both are well defined by standardization units and organizations such as IHE (Integrating the Healthcare Enterprise) .

Based on the data gathered in health problem assessment and the expert identification, appointment options with available time slots can be presented to the patient. Based on various ranking algorithms appointment options for appointments with a suitable healthcare professional, in particular with a suitable physician, are presented to the patient, e.g. via a user interface of a portable or non-portable user terminal of the patient.

To guide a patient through its journey the system can detect automatically based on the patient's data in the system which healthcare service provider is best able to provide the service such as a medical consultation and if it is even feasible. A set of functionalities is provided by the system ranging from an online presence, to appointment booking, task handling, reminders and data population. While the patient portal according to the present invention guides the patient through the progress of arranging his appointments the healthcare service provider benefits from a well-informed, prepared patient as well as a valid appointment including all necessary data for conducting the appointment successfully.

The patient portal according to the present invention can comprise a browser-based web application that provides patients with a secure, facility-wide access to their electronic health data and records. It also enables them to participate meaningfully in decision-making by sharing up-to-date medical information with the patients. The patient portal eliminates the inconvenience of excessive documentation for patients, relieves care teams of the burden of document organization, and keeps all documentation secure. The participating patients and healthcare service providers can be authorized by the system to participate in the distributed system. The data is preferably transmitted in the distributed system in encrypted form.

The patient portal according to the present invention further enables well-coordinated healthcare delivery and interactions. It drives less readmissions and referrals to other participating healthcare service providers.

The different embodiments described above can also be combined with each other.

Although the present invention has been described above on the basis of preferred embodiments it is not restricted to these, but can be modified in different ways.

For instance, the location of a portable user equipment of a user can be tracked by the healthcare system to give the patient directions to find a healthcare service provider in his vicinity. For example, a patient in a hospital can be guided to a first appointment with a radiologist located in the basement of the hospital to perform a diagnostic scan of his body and to generate patient image data. Then the investigated patient is redirected to the next appointment with a physician located in another floor of the hospital requiring the patient image data for the treatment of the patient.

Further, a ranking algorithm can be installed to calculate a ranking of the patients and a ranking of the healthcare service providers depending on a task fulfillment rate. This provides a motivation for patients to provide all necessary data for an appointment with a healthcare service provider as soon as possible. Diligent patients with a high ranking may get an appointment sooner than other patients who do not provide their necessary patient data in time.

Although the present invention has been described in the above by way of embodiments, it is not limited thereto, but rather can be modified in a wide range of ways. In particular, the invention can be changed or modified in various ways without deviating from the core of the invention.

Independent of the grammatical usage, individuals with male, female or other gender identities are included within the term.

## Claims

1. A computer-implemented method for providing data of a patient used in a scheduled appointment with a healthcare service provider, wherein the method comprises:
determining (S1) patient data required for the scheduled appointment depending on an appointment type of the scheduled appointment;
calculating (S2) missing patient data depending on the determined patient data required for the scheduled appointment and depending on available patient data of the respective patient stored in an electronic health record (EHR) of the patient;
determining(S3) at least one data input channel suitable for transfer of the calculated missing patient data;
requesting (S4) a transfer of the calculated missing patient data from a terminal device of the patient and/or from a terminal device of another healthcare service provider via the determined data input channel; and
receiving (S5) from the terminal device of the patient and/or from the terminal device of the other healthcare service provider the requested missing patient data transferred via the determined data input channel.

2. The computer-implemented method according to claim 1, wherein the data input channel comprises a document submission channel configured to submit documents, a patient input channel configured to collect patient data input by a patient or by a healthcare professional into a form or into a patient questionnaire displayed on a user interface of a terminal device and/or comprises a monitoring device input channel configured to collect health related patient data provided by a health monitoring device.

3. The computer-implemented method according to any of the preceding claims, wherein the missing patient data received(S5) via the determined data input channel is stored in the electronic health record (EHR) of the patient.

4. The computer-implemented method according to any of the preceding claims, wherein a data provision task is generated automatically for a patient and/or for another healthcare service provider to provide the missing patient data of the patient required for the scheduled appointment and wherein the generated data provision task is then transmitted to a terminal device of the patient or to a terminal device of the healthcare service provider.

5. The computer-implemented method according to any of the preceding claims, wherein the calculated missing patient data comprises missing patient data available to the patient and missing patient data to be provided by another healthcare service provider.

6. The computer-implemented method according to claim 5, wherein a data provision task is triggered automatically if the calculated missing patient data comprises missing patient data to be provided by another healthcare service provider.

7. The computer-implemented method according to claim 6, wherein the missing patient data is received from a terminal device of the healthcare service provider via a professional data input channel with a high bandwidth and/or with a data security mechanism in response to the triggered data provision task.

8. The computer-implemented method according to any of the claim 5 - 7, wherein a terminal device of the other healthcare service provider evaluates the received data provision task to schedule automatically an appointment with the respective patient for generation of the missing patient data if necessary.

9. The computer-implemented method according to claim 4, wherein a re-scheduling of the appointment is performed automatically if the data provision task is not fulfilled before the scheduled appointment.

10. The computer-implemented method according to any of the preceding claims, wherein the appointment is setup and booked by a participating user including the patient or the healthcare service provider or wherein the appointment is setup automatically in response to an observed event, in particular in response to a critical condition of the patient, wherein the critical condition is detected automatically based on patient data collected from a health monitoring device or indicated by a healthcare professional observing the patient.

11. A patient portal comprising a server adapted to perform the computer-implemented method according to any of the preceding claims 1 to 10.

12. The patient portal according to claim 11, wherein the server of the patient portal is connected via at least one data input channel to a user terminal device of the patient and/or to at least one monitoring device adapted to monitor the patient.

13. The patient portal according to claim 11 or 12, wherein the server of the patient portal is connected via at least one professional data input channel with a high bandwidth and/or with a data security mechanism to a plurality of distributed terminal devices of healthcare service providers.

14. The patient portal according to any of the claims 11 to 13, wherein the scheduled appointment of the patient with a healthcare service provider comprises a virtual online appointment or a personal appointment.

15. The patient portal according to any of the claims 11 to 14, wherein the server of the patient portal has access to a database storing an electronic health record (EHR) of the patient.
